# EUROPEAN PATENT APPLICATION

(11) **EP 2 130 528 A1**
(43) Date of publication of application: **09.12.2009**
(21) Application number: 08711740.4
(22) Date of filing: 21.02.2008
(51) Int. Cl.: A61K 8/22, A61K 8/19, A61K 8/24, A61K 8/38, A61K 8/49, A61Q 11/00

(54) **TOOTH-BLEACHING MATERIAL AND METHOD OF BLEACHING TOOTH**

(30) Priority: 23.03.2007 JP 2007075822
(71) Applicant: Mitsubishi Gas Chemical Company, Inc., Chiyoda-ku Tokyo 100-8324 (JP)
(72) Inventor: KURATA, Hiroshi, Tokyo 125-0051 (JP); KUMAGAI, Yuki, Tokyo 125-0051 (JP); KIMIDUKA, Kenichi, Tokyo 125-0051 (JP); KONDO, Yoshinori, Tokyo 125-0051 (JP); KAWABATA, Yasunari, Tokyo 125-0051 (JP)
(74) Representative: Müller-Boré & Partner Patentanwälte
(86) International application number: PCT/JP2008/052957
(87) International publication number: WO 2008/117598

(57) **Abstract**

A tooth bleaching material that is safe and has good operability and high bleaching effect and a tooth bleaching method using the tooth bleaching material are provided by bleaching and removing colored sediments (colored or discolored deposits) on a tooth with a tooth bleaching material containing hydrogen peroxide and/or a compound generating hydrogen peroxide in an aqueous solution, and a dicarboxylic anhydride, and also containing a basic compound and a thickening agent, and a tooth bleaching method containing attaching the tooth bleaching material to a surface of a discolored tooth after dilution or without dilution irradiating with light.

## Description

### [Technical Field]

The present invention relates to a bleaching material for bleaching and removing colored sediments (colored or discolored deposits) on a tooth, and a method for bleaching a tooth.

### [Background Art]

In recent years, there has been an increasing demand for esthetic improvements of teeth such as improvements of contours, alignment and integrity of teeth in dental therapy, and in particular, there have been more cases of young women desiring a dental therapy for whitening teeth as an important element of beauty.

A bleaching material for a tooth requires such conditions that include: (a) pronounced bleaching results, (b) non-toxicity of the bleaching material used, (c) convenient operations, (d) no deterioration in dental physical properties after bleaching, (e) effectiveness to both vital tooth bleaching and non-vital tooth bleaching, and (f) bleaching results obtained in a short time. However, in conventional bleaching materials, the primary bleaching agent is a high concentration hydrogen peroxide, resulting in problems in terms of safety and convenience.

In view of the issues above, as a bleaching material that is excellent in safety and convenience and exhibits pronounced results in a short time, a bleaching material using a combination of titanium dioxide having a photocatalytic action and a low concentration aqueous hydrogen peroxide is proposed (see Patent Document 1). A bleaching material containing calcium phosphate compound, a fluorine compound, a peroxide compound and an acid, such as citric acid and the like is proposed (Patent Document 2).
[Patent Document 1] JP-A-11-92351
[Patent Document 2] JP-A-11-116421

### [Disclosure of the Invention]

### [Problems to be solved by the Invention]

An object of the present invention is to provide a tooth bleaching material that is safe and convenient for use and has high bleaching effect.

### [Means for solving the Problems]

As a result of earnest investigations made by the inventors, it has been found that such a tooth bleaching material exhibits excellent bleaching effect and is excellent in safety that contains hydrogen peroxide and/or a compound generating hydrogen peroxide in an aqueous solution, and a dicarboxylic anhydride, which forms an organic peroxide through reaction with the compounds, and thus the present invention has been completed. Accordingly, the present invention relates to a tooth bleaching material containing hydrogen peroxide and/or a compound generating hydrogen peroxide in an aqueous solution, and a dicarboxylic anhydride.

### [Best Mode for carrying out the Invention]

The present invention will be described in detail below.
Examples of the compound generating hydrogen peroxide in an aqueous solution include a percarbonate salt, a perborate salt, a perphosphate salt, a persulfate salt, calcium peroxide, magnesium peroxide, carbamide peroxide and the like, at least one of which may be used, and the tooth bleaching material of the present invention preferably contains hydrogen peroxide and a dicarboxylic anhydride.
Examples of the dicarboxylic anhydride include succinic anhydride, methylsuccinic anhydride, glutaric anhydride, 3-methylglutaric anhydride, maleic anhydride, cis-1,2-cyclohexanedicaroxylic acid and the like, preferably succinic anhydride, methylsuccinic anhydride, 3-methylglutaric anhydride, maleic anhydride and cis-1,2-cyclohexanedicaroxylic acid, and more preferably succinic anhydride and methylsuccinic anhydride.
The tooth bleaching material of the present invention preferably further contains a basic compound, and examples of the basic compound include sodium hydroxide, potassium hydroxide, calcium hydroxide, sodium pyrophosphate, potassium pyrophosphate, sodium polyphosphate, potassium polyphosphate, trisodium phosphate and tripotassium phosphate, and preferably sodium hydroxide, sodium pyrophosphate and sodium polyphosphate, and at least one selected from them may be used.

The tooth bleaching material of the present invention preferably contains a thickening agent for providing such a viscosity that is sufficient for coating on a surface of a tooth. Inorganic and organic thickening agents may be used, and they may be used in combination.

Examples of the inorganic thickening agent include titanium oxide, calcium silicate, magnesium silicate, magnesium sodium silicate, silica powder, fumed silica, an inorganic clay mineral and the like, and an inorganic clay mineral is preferably used. Examples of the inorganic clay mineral include dickite, nacrite, kaolinite, anorthite, halloysite, metahalloysite, chrysotile, lizardite, serpentine, antigorite, beidellite, montmorillonite, sauconite, stevensite, nontronite, saponite, hectorite, vermiculite, smectite, sepiolite, illite, sericite, glauconite-montmorillonite, roselite-montmorillonite, chlorite-vermiculite, illite-montmorillonite, halloysite-montmorillonite, kaolinite-montmorillonite and the like. Among the inorganic clay minerals, a layered structure type clay mineral, such as montmorillonite, sauconite, smectite, stevensite, beidellite, nontronite, saponite, hectorite, vermiculite and the like, is preferably used. This is because a layered structure type clay mineral is swellable by incorporating water molecules between the unit layers in the structure thereof. The inorganic clay mineral may be a natural product or a synthetic product. Examples of the synthetic product include synthetic magnesium sodium lithium silicate (laponite). A mixture of two or more of them may be used.

Examples of the organic thickening agent include, a polyhydric alcohol, such as glycerin, diglycerin, ethylene glycol, diethylene glycol, propylene glycol, dipropylene glycol, sorbitol, mannitol, polyethylene glycol, polypropylene glycol, polyoxyethylene polyoxypropylene glycol and the like, starch, starch sodium glycolate, starch sodium phosphate, galactomannan, cellulose nitrate, methyl cellulose, carboxymethyl cellulose., sodium carboxylmethyl cellulose, hydroxymethyl cellulose, hydroxyethyl cellulose, hydroxypropyl cellulose, pectic acid, pectinic acid, cellulose sodium glycolate, sodium alginate, agar, carrageenin, proteoglycan, glycoprotein, gelatin, actin, tubulin, hemoglobin S, insulin, fibrin, egg albumin, serum albumin, myosin, collagen, a polypeptide compound, casein, sodium polyacrylate, polyvinyl alcohol, polyethylene oxide, polyacrylamide, a carboxyvinyl polymer, polyvinylpyrrolidone, a methyl vinyl ether-maleic anhydride copolymer and the like, and preferred examples among these include sodium polyacrylate, sodium alginate, methyl cellulose, carboxymethyl cellulose, sodium carboxylmethyl cellulose, hydroxymethyl cellulose, hydroxyethyl cellulose, a carboxyvinyl polymer and the like. These may be a natural product or a synthetic product, and a mixture of two or more of them may be used.

In the tooth bleaching material of the present invention, the amount of the hydrogen peroxide and/or the compound generating hydrogen peroxide in an aqueous solution mixed is from 0.05 to 80% by mass, and preferably from 0.1 to 40% by mass, and the amount of the dicarboxylic anhydride mixed is from 0.05 to 40% by mass, and preferably from 0.1 to 30% by mass. The mixing temperature is not particularly limited and may be ordinary temperature. The mixing time is from 0.02 minute to 1 week, and preferably from 0.02 to 30 minutes. An organic peroxy acid can be generated in a short time by mixing hydrogen peroxide and/or the compound generating hydrogen peroxide in an aqueous solution with the dicarboxylic anhydride.

The calculating method of the concentration of hydrogen peroxide and the concentration of an organic peroxy acid will be described. A potassium permanganate solution is added to an organic peroxy acid solution until pink color of permanganic acid does not disappear to decompose hydrogen peroxide contained in the organic peroxy acid solution, thereby providing the concentration of hydrogen peroxide. Subsequently, the concentration of peroxycarboxyl groups is measured by iodometry, thereby providing the concentration of the organic peroxy acid.

The tooth bleaching material of the present invention preferably has a pH value of from 3 to 8. In the case where the pH is less than 3, the bleaching effect may be lowered, and in the case where it exceeds 8, decomposition of the organic peroxy acid may be accelerated to make maintenance of the prescribed concentration difficult. Accordingly, the tooth bleaching material of the present invention preferably contains a basic compound, and the amount of the compound mixed is from 0.05 to 30% by mass, and preferably from 0.1 to 10% by mass.

The tooth bleaching material of the present invention may contain a water-miscible solvent, such as an alcohol compound and the like, a perfume, a colorant, a hyperesthesia preventing agent, such as potassium nitrate and the like, and other additives, for improving operability.

The tooth bleaching method of the present invention contains mixing hydrogen peroxide and/or a compound generating hydrogen peroxide in an aqueous solution with a dicarboxylic anhydride, and coating the resulting tooth bleaching material on a surface of a tooth after dilution or without dilution. Water may be used for diluting the tooth bleaching material of the present invention.

The tooth bleaching material of the present invention is coated on a surface of a tooth, which is irradiated with light, thereby the bleaching effect can be further enhanced. The light to be radiated is preferably light having such a wavelength that is less harmful to a human body. Examples of the light include light containing components having a wavelength of 300 nm or more, and light containing components having a wavelength of from 380 to 500 nm is preferred. Examples of a light source used include an incandescent lamp, a fluorescent lamp, a halogen lamp, a black-light lamp, a metal halide lamp, a xenon lamp, a mercury lamp, an UV lamp, an LED (light-emitting diode), and a laser sighting device, such as a semiconductor laser and the like. An unnecessary wavelength range is cut from the light emitted from the light source with a suitable filter, thereby providing light having a desired wavelength. The number of times of coating of the bleaching material and radiation of light may be appropriately adjusted depending on the extent of discoloration of a tooth.

### [Example]

The present invention will be described specifically with reference to examples below, but the present invention is not limited to the examples.

### Examples 1 to 9

The second agents were added to the first agents as shown in Table 1, and mixed for a prescribed period of time, thereby providing tooth bleaching materials obtained therefrom.

**Table 1**

| Example | First agent | | | Second agent | | Mixing time | Bleaching material | | |
|---|---|---|---|---|---|---|---|---|---|
| | Hydrogen peroxide or compound generating hydrogen peroxide in aqueous solution | | Water | Dicarboxylic anhydride | | | Organic peroxy acid | Hydrogen peroxide | pH |
| | | (g) | (g) | | (g) | (min) | (% by mass) | (% by mass) | |
| 1 | 35% aqueous hydrogen peroxide | 50.9 | - | succinic anhydride | 5.0 | 130 | 12 | 29 | 2 |
| 2 | 35% aqueous hydrogen peroxide | 45.2 | - | glutaric anhydride | 25.0 | 350 | 2 | 22 | 2 |
| 3 | 35% aqueous hydrogen peroxide | 4.5 | - | methylsuccinic anhydride | 1.0 | 10 | 17 | 24 | 2 |
| 4 | 35% aqueous hydrogen peroxide | 4.5 | - | 3-methylglutaric anhydride | 2.0 | 10 | 7 | 20 | 2 |
| 5 | 35% aqueous hydrogen peroxide | 4.5 | - | 3-methylglutaric anhydride | 2.0 | 8,650 | 31 | 17 | 2 |
| 6 | 35% aqueous hydrogen peroxide | 4.5 | - | maleic anhydride | 1.6 | 3 | 29 | 19 | 1 |
| 7 | 3.5% aqueous hydrogen peroxide | 10.1 | - | succinic anhydride | 0.7 | 60 | 3 | 3 | 2 |
| 8 | 3.5% aqueous hydrogen peroxide | 10.1 | - | methylsuccinic anhydride | 0.8 | 5 | 2 | 3 | 2 |
| 9 | carbamide peroxide | 1.0 | 10.0 | succinic anhydride | 0.7 | 270 | 4.2 | 2.3 | 2 |

### Example 10

The tooth bleaching material obtained in Example 3 was diluted with purified water and adjusted for pH with NaOH, thereby providing a tooth bleaching material having a total active oxygen amount of 1.3% (methyl peroxysuccinic acid: 1.8% by mass, hydrogen peroxide: 2.3% by mass) and pH 5.

### Example 11

The tooth bleaching material obtained in Example 7 was adjusted for pH with a NaOH aqueous solution, thereby providing a tooth bleaching material having a total active oxygen amount of 1.1% (peroxysuccinic acid: 1.9% by mass, hydrogen peroxide: 1.9% by mass) and pH 6.

### Comparative Example 1

3% by mass aqueous hydrogen peroxide (active oxygen amount: 1.4%) adjusted to pH 6 was prepared.

### Comparative Example 2

An aqueous solution containing 1.8% by mass of methyl succinic acid and 2.3% by mass of hydrogen peroxide adjusted to pH 5 was prepared.

### Comparative Example 3

An aqueous solution containing 1.9% by mass of succinic acid and 1.1% by mass of hydrogen peroxide adjusted to pH 6 was prepared.

### Comparative Example 4

30% by mass aqueous hydrogen peroxide (active oxygen amount: 14%) adjusted to pH 3 was prepared. It had high dermal irritancy.

### Example 12

The same procedures as in Example 11 were carried out except that sodium polyacrylate was added for thickening upon preparing the tooth bleaching material, thereby providing a tooth bleaching material containing 1.9% by mass of peroxysuccinic acid, 1.0% by mass of hydrogen peroxide and 1.0% by mass of sodium polyacrylate and having pH 6.0. It became a viscous composition and had property that was easily coated on a surface of a tooth.

### Example 13

The same procedures as in Example 11 were carried out except that glycerin and a carboxyvinyl polymer were added for thickening upon preparing the tooth bleaching material, thereby providing a tooth bleaching material containing 1.9% by mass of peroxysuccinic acid, 1.0% by mass of hydrogen peroxide, 30% by mass of glycerin and 1.0% by mass of the carboxyvinyl polymer and having pH 6.0. It became a viscous composition and had property that was easily coated on a surface of a tooth and was hard to be dried.

For examining the resulting tooth bleaching materials for bleaching effect, (A) a model bleaching test with hematoporphyrin dyed paper and (B) a bleaching test of a discolored tooth were performed.

### (A) Hematoporphyrin Model Bleaching Test

The hematoporphyrin model bleaching test was performed in the following manner.
(1) Glossy paper was dyed with a hematoporphyrin solution.
(2) Dyed paper having values of L* (brightness), a* (redness) and b* (yellowness) close to 64, 15 and 25, respectively, was selected.
(3) The bleaching materials each were coated on the dyed paper.
(4) The test was performed for each cases where light was irradiated and light was not irradiated.

### (4-1) Light radiated

(1) The coated surface was irradiated with light by using a dental visible light radiating apparatus (Optilux 501, a trade name, produced by Demetron, Inc.).
(2) The dyed paper after the total light radiation time of 1 minute was measured for L*, a* and b* with a spectral colorimeter (SE-2000, produced by Nippon Denshoku Industries, Co., Ltd.).
(3) The differences (Δ) of L*, a* and b* before the test and after the total light radiation time of 1 minute were calculated as ΔL*, Δa* and Δb*, respectively.

### (4-2) Light not radiated

(1) The coated dyed paper was allowed to stand in a dark space.
(2) After lapsing 3 hours, the dyed paper was taken out from the dark space and measured for L*, a* and b* with a spectral colorimeter (SE-2000, produced by Nippon Denshoku Industries, Co., Ltd.).
(3) The differences (Δ) of L*, a* and b* before the test and after lapsing 3 hours were calculated as ΔL*, Δa* and Δb*, respectively.
   The results of the hematoporphyrin model bleaching test using the bleaching materials prepared in Examples 10 and 11 and Comparative Examples 1 to 4 are shown in Table 2.

### (B) Bleaching Test of Discolored Tooth

The bleaching test of a discolored tooth (extracted tooth) was performed in the following manner.
(1) As a pretreatment, plaque, tartar, tar and the like are removed from the tooth using an ultrasonic wave scaler.
(2) The surface of the tooth was cleaned by an ordinary method with a rubber cup or the like, and then dried.
(3) The tooth was subjected to a simple moisture-proof treatment.
(4) The bleaching material was coated on the surface of the tooth, which was irradiated with light using a dental visible light radiating apparatus (Optilux 501, a trade name, produced by Demetron, Inc.).
(5) After radiating light for 2 minutes, the bleaching material was newly coated, and the surface of the tooth was irradiated with light, every time. The operation was repeated 10 times (total irradiation time: 20 minutes).
The surface of the tooth after bleaching was measured for color with a spectral colorimeter (SE-2000, produced by Nippon Denshoku Industries, Co., Ltd.), and the change of the discolored tooth was expressed by L* (brightness), a* (redness) and b* (yellowness). The results of the bleaching test of a discolored tooth using the bleaching materials prepared in Examples 10 and 11 and Comparative Examples 1 to 4 are shown in Table 3.

**Table 2**

| | Measurement results of color difference | | | | | |
|---|---|---|---|---|---|---|
| | Light radiated | | | Light not radiated | | |
| | ΔL* | Δa* | Δb* | ΔL* | Δa* | Δb* |
| Example 10 | 19 | -15 | -13 | 9 | -10 | -2 |
| Example 11 | 26 | -16 | -15 | 14 | -12 | -4 |
| Comparative Example 1 | 1 | -8 | -8 | -2 | -3 | -5 |
| Comparative Example 2 | 0 | -8 | -6 | -1 | -3 | -3 |
| Comparative Example 3 | 0 | -7 | -5 | -2 | -3 | -3 |
| Comparative Example 4 | 7 | -9 | -10 | 2 | -5 | -10 |

| | | | | | | |
|---|---|---|---|---|---|---|
| ΔL* = L* after test - L* before test (larger value: increased brightness) Δa* = a* after test - a* before test (smaller value: decreased redness) Δb* = b* after test - b* before test (smaller value: decreased yellowness) | | | | | | |

**Table 3**

| | Color difference | | | | | |
|---|---|---|---|---|---|---|
| | Before bleaching | | | After irradiating light for 20 minutes | | |
| | L* | a* | b* | L* | a* | B* |
| Example 10 | 63 | -3 | 6 | 64 | -2 | 3 |
| Example 11 | 62 | -3 | 4 | 63 | -2 | 1 |
| Comparative Example 1 | 58 | -3 | -1 | 58 | -3 | -1 |
| Comparative Example 2 | 63 | -2 | 3 | 64 | -2 | 3 |
| Comparative Example 3 | 60 | -3 | 2 | 60 | -3 | 2 |
| Comparative Example 4 | 60 | -2 | -1 | 63 | -2 | -1 |

### Examples 14 to 22

The first agents and the second agents shown in Table 4 were mixed to prepare basic hydrogen peroxide solutions, to which the third agents were added and mixed for a prescribed period of time, thereby providing tooth bleaching materials obtained therefrom.

**Table 4**

| Example | First agent | | Second agent | Third agent | | Mixing time | Bleaching material | | |
|---|---|---|---|---|---|---|---|---|---|
| | Hydrogen peroxide | | NaOH | Dicarboxylic anhydride | | | Organic peroxy acid | Hydrogen peroxide | pH |
| | | (g) | (g) | | (g) | (min) | (% by mass) | (% by mass) | |
| 14 | 3.4% aqueous hydrogen peroxide | 13.1 | 0.5 | succinic anhydride | 1.3 | 5 | 10.9 | 0.2 | 5 |
| 15 | 3.1% aqueous hydrogen peroxide | 14.5 | 0.2 | succinic anhydride | 0.4 | 5 | 3.4 | 2.2 | 5 |
| 16 | 3.0% aqueous hydrogen peroxide | 14.8 | 0.1 | succinic anhydride | 0.1 | 5 | 2.8 | 1.3 | 5 |
| 17 | 5.5% aqueous hydrogen peroxide | 40.7 | 0.8 | succinic anhydride | 0.9 | 3 | 15.0 | 10.0 | 5 |
| 18 | 5.6% aqueous hydrogen peroxide | 11.9 | 0.1 | methylsuccinic anhydride | 0.2 | 1 | 2.0 | 4.9 | 5 |
| 19 | 5.6% aqueous hydrogen peroxide | 11.9 | 0.1 | glutaric anhydride | 0.2 | 1 | 1.7 | 4.8 | 5 |
| 20 | 5.6% aqueous hydrogen peroxide | 11.9 | 0.1 | 3-methylglutaric anhydride | 0.3 | 1 | 1.9 | 4.9 | 4 |
| 21 | 5.6% aqueous hydrogen peroxide | 11.9 | 0.2 | cis-1,2-cyclohexane- dicarboxylic anhydride | 0.3 | 5 | 2.4 | 4.8 | 4 |
| 22 | 5.6% aqueous hydrogen peroxide | 11.9 | 0.1 | maleic anhydride | 0.2 | 1 | 0.4 | 5.3 | 4 |

### Example 23

2.1 g of sodium percarbonate was dissolved in 10.5 g of pure water. The solution had pH 10. 2. 6 g of 3-methylglutaric anhydride was added to the sodium percarbonate aqueous solution, and mixed for 3 minutes, thereby providing a tooth bleaching material. The resulting tooth bleaching material had a concentration of peroxy 3-methylglutaric acid of 4.7% by mass and a concentration of hydrogen peroxide of 0% by mass.

### Example 24

1.4 g of sodium percarbonate was dissolved in 10.0 g of pure water. The solution had pH 10. 1.5 g of phthalic anhydride was added to the sodium percarbonate aqueous solution, and mixed for 60 minutes, thereby providing a tooth bleaching material. The resulting tooth bleaching material had a concentration of peroxy phthalic acid of 3.5% by mass and a concentration of hydrogen peroxide of 0% by mass.

### Example 25

The same procedures as in Example 15 were carried out except that sodium polyacrylate was added to the first agent upon preparing the tooth bleaching material, thereby providing a tooth bleaching material containing 3.0% by mass of peroxysuccinic acid, 2.0% by mass of hydrogen peroxide and 1.0% by mass of sodium polyacrylate and having pH 5. It became a viscous composition and had property that was easily coated on a surface of a tooth.

### Example 26

The same procedures as in Example 15 were carried out except that glycerin and a carboxyvinyl polymer were added to the first agent upon preparing the tooth bleaching material, thereby providing a tooth bleaching material containing 2.8% by mass of peroxysuccinic acid, 2.0% by mass of hydrogen peroxide, 30% by mass of glycerin and 1.0% by mass of the carboxyvinyl polymer and having pH 6.0. It became a viscous composition and had property that was easily coated on a surface of a tooth and was hard to be dried.

For examining the resulting tooth bleaching materials of the present invention for bleaching effect, (A) the model bleaching test with hematoporphyrin dyed paper and (B) the bleaching test of a discolored tooth were performed. The results of the hematoporphyrin bleaching test using the bleaching materials prepared in Examples 15 and 17 are shown in Table 5, and the results of the bleaching test of a discolored tooth using the same are shown in Table 6.

**Table 5**

| | Measurement results of color difference | | | | | |
|---|---|---|---|---|---|---|
| | Light radiated | | | Light not radiated | | |
| | ΔL* | Δa* | Δb* | ΔL* | Δa* | Δb* |
| Example 15 | 26 | -15 | -20 | 18 | -13 | -6 |
| Example 17 | 30 | -16 | -23 | 28 | -17 | -17 |

**Table 6**

| | Color difference | | | | | |
|---|---|---|---|---|---|---|
| | Before bleaching | | | After irradiating light for 20 minutes | | |
| | L* | a* | b* | L* | a* | b* |
| Example 15 | 61 | -2 | 9 | 65 | -2 | 5 |
| Example 17 | 58 | -3 | 8 | 61 | -2 | 3 |

## Claims

1. A tooth bleaching material comprising hydrogen peroxide and/or a compound generating hydrogen peroxide in an aqueous solution, and a dicarboxylic anhydride.

2. The tooth bleaching material according to claim 1 further comprising a basic compound.

3. The tooth bleaching material according to claim 1 comprising hydrogen peroxide and a dicarboxylic anhydride.

4. The tooth bleaching material according to claim 1, wherein the dicarboxylic anhydride is at least one selected from succinic anhydride, methylsuccinic anhydride, 3-methylglutaric anhydride, maleic anhydride and cis-1,2-cyclohexanedicaroxylic acid.

5. The tooth bleaching material according to claim 2, wherein the basic compound is at least one selected from sodium hydroxide, potassium hydroxide, calcium hydroxide, sodium pyrophosphate, potassium pyrophosphate, sodium polyphosphate, potassium polyphosphate, trisodium phosphate and tripotassium phosphate.

6. The tooth bleaching material according to claim 1 further comprising a thickening agent.

7. A tooth bleaching method comprising: mixing hydrogen peroxide and/or a compound generating hydrogen peroxide in an aqueous solution with a dicarboxylic anhydride; and coating a resulting tooth bleaching material on a surface of a tooth after dilution or without dilution.

8. The tooth bleaching method according to claim 7, wherein the tooth bleaching material is coated on a surface of a tooth, which is irradiated with light.
